# EUROPEAN PATENT SPECIFICATION

(11) **EP 2 153 785 B1**
(45) Date of publication and mention of the grant of the patent: **26.12.2012**
(21) Application number: 08014378.7
(22) Date of filing: 12.08.2008
(51) Int. Cl.: A61B 17/70, B26D 3/14, B26D 3/16, B26F 1/00, B26F 1/12, A61B 17/88

(54) **Flexible stabilization device including a rod and tool for manufacturing the rod**
Flexible Stabilisierungsvorrichtung mit einer Stange und einem Werkzeug zur Herstellung der Stange
Dispositif de stabilisation flexible incluant une tige et outil de fabrication de la tige

(43) Date of publication of application: 17.02.2010
(60) Divisional application: 12158902.2
(73) Proprietor: Biedermann Technologies GmbH & Co. KG, 78166 Donaueschingen (DE)
(72) Inventor: Biedermann, Lutz, 78048 VS-Villingen (DE); Matthis, Wilfried, 79367 Weisweil (DE); Pabst, Martin, 78166 Donaueschingen (DE); Dannecker, Berthold, 78112 St. Georgen (DE)
(74) Representative: Prüfer & Partner GbR European Patent Attorneys

(56) References cited:
- US-A- 4 281 546
- US-A- 4 784 638
- US-A1- 2004 161 310
- US-A1- 2005 261 686
- US-A1- 2007 093 814
- US-A1- 2007 225 710
- US-A1- 2007 299 442
- US-A1- 2008 177 320

## Description

The invention relates to a tool for manufacturing a flexible stabilization device for connecting at least two bone anchoring devices which are attached to vertebrae of the spinal column.

Such flexible stabilization devices may generally comprise a rod which is provided with a considerable degree of stiffness in order to stabilize the spinal column. The bone anchoring devices may include receiving parts, which are provided with recesses to receive the rod, and fixation screws to accomplish a tight connection between the receiving parts and the rod. The receiving parts are further connected with, or integrally formed with bone screws, which may be screwed in to the adjacent vertebrae, e.g., pedicles. Thereby, multiple anchoring devices may be connected using the rod as described above.

In recent years there had been many efforts to provide a connection rod with flexible behaviour. The flexibility allows the spinal column to be moved in a controlled manner.

As an example, WO 1996/016608 A1 shows a vertebral instrumentation rod which is made of a basically rigid material, i.e., a metal or metal alloy. Hence, the rod is rigid in a first part where a cross section is generally cylindrical. However, in a second part, the rod is substantially flat to allow for flexion in a sagittal plane while impeding flexion in the frontal plane. In a transition zone, the degree flatness smoothly increases from the first towards the second part.

US 2005/085815 A1, by Applicant, shows a rod-shaped element wherein an flexible section is integrally formed with two adjacent rigid sections. The rod-shaped element may be composed of a metal material, and the flexible section may be provided by a coil spring allowing for flexion. In one specific embodiment, a cross section of the flexible section, i.e., of the coil spring, is flattened in one direction in achieve desired flexion properties.

US 2007/049937 A1, by Applicant, shows in one embodiment a rod-shaped implant element wherein an flexible section is connected between stiff portions. The flexible section is made from, e.g., polyurethane or polysiloxane, whereas the stiff portions are made from, e.g., titanium. The connection is provided by threads. Further, the flexible section may have a reduced or increased thickness depending on a desired amount of compression or extension capabilities.

US 2007/270821 A1 shows a vertebral stabilizer, which includes a connector formed as a single piece construction. The connector has an annular section of a reduced diameter, as compared with interposed sections, in order to enable stretching of the same. The connector may be formed from flexible fiber material.

US 2003/191470 A1 shows a dynamic fixation system wherein a rod may be shaped and thinned to function as a spring or pivot. The rod may be connected with pedicle screws via connectors while elastomer materials may be used for the rod, although not being preferred. A stepped flattened cross sectional profile may be obtained depending on the desired flexion or torsion characteristics.

US 2007/0225710 A1 discloses a spinal stabilization device. In one embodiment of a flexible tubular rod, the rod may be fabricated - among others - from an elastomer. The rod has a middle portion with a surface in which spiral grooves are formed. In another embodiment disclosed therein, a flexible rod is provided with transverse holes or tunnels drilled therethrough.

US 4,784,638 discloses an apparatus for making an angled hole ventricular catheter. The apparatus comprises a holding apparatus and a handle of a cutting apparatus provided with multiple rod-like cutting elements which mate with respective holes of the holding apparatus. The holes open towards a longitudinally extending aperture into which the catheter may be inserted for piercing holes into the same by means of the cutting elements.

It is an object of the invention to further improve known techniques of stabilizing human vertebrae and to provide a flexible stabilization device that covers desired flexion and torsion characteristics while reducing the efforts and costs of manufacturing the same.

These and other objects are solved by a tool for separating material from the rod according to claim 1. Further aspects and embodiments become apparent from the appended claims.

The recess formed thereby in the elastomer rod may influence the local bending and/or torsional properties of the rod. In one aspect, the separation may yield a directed removal of material from the rod along a first direction. Hence, the recess formed accordingly may affect the flexural rigidity of the rod in one particular (second) direction, while the original flexural rigidity of the rod may be maintained in another direction, e.g., perpendicular to the first direction.

Therefore, the recess applied to the rod may serve to achieve a reduced flexural rigidity of the rod along an orientation direction according to the specific needs of the concerned spinal column. For example, the overall flexural rigidity of the rod may be reduced at a specific position along the spinal column in order to allow for an improved flexion in, e.g., the frontal or the sagittal plane.

The separation process refers to punching out material from the rod using a punching or cutting tool as specified in claim 1. This further allows implementing multiple punched recesses to be formed along the rod surface according to the desired needs - under consideration of local flexural strength and its orientation with respect to the rod, or spinal column. According to specific variations of this aspect, the depth, length, mutual orientation or the number density of the multiple recesses can be varied along the rod to treat different parts of the spinal column in the most appropriate manner regarding flexion and/or torsion.

A tool according to a related aspect includes a socket die with a first bore for receiving the rod connector, and at least one second bore for receiving a first punching press, and further includes the punching press, which fits within the at least one second bore being moveable with respect thereto. Thereby, the first and the at least one second bore intersect each other off-center from the longitudinal axis of the first bore to enable punching out material from the flexible rod to form the recess due to the movable punching press.

One particular advantage of using a rod having punched recesses and employing a corresponding punching tool as described above may be found in that the shaping of the rod can be accomplished by the surgeon or another person prior to the actual surgery, since no complex injection molding method is required. The tool can also be used easily in the operating room environment.

Further features and advantages of the bone anchoring device will become apparent and will be best understood by reference of the following detailed description taken in conjunction with the accompanying drawings. Therein:
- Fig. 1: shows a schematic drawing of the spinal column, to which is added a stabilization device including a set of bone screws and one of three types of rods;
- Fig. 2: shows a rod including recesses in a perspective view;
- Fig. 3: same as Fig. 2, but in a top view, the rod being rotated by 90 degrees between the states shown;
- Fig. 4: a perspective view of a tool for separating material from an elastomer rod according to a first embodiment in a state before separation;
- Fig. 5: same as in Fig. 4, but in a state when the tool is operated to separate the material;
- Fig. 6: same as in Fig. 4, but in a cross-sectional representation;
- Fig. 7: same as in Fig. 5, but in a cross-sectional representation;
- Fig. 8: in a perspective view a second embodiment of a tool for separating material from an elastomer rod, wherein the state shown refers a first step of a separation method;
- Fig. 9: same as Fig. 8, but with reference to a second step;
- Fig. 10: same as Fig. 8, but with reference to a third step;
- Fig. 11: same as Fig. 8, but with reference to a fourth step;
- Fig. 12: shows further examples of recesses which may be implemented with a flexible rod by means of a separation method.

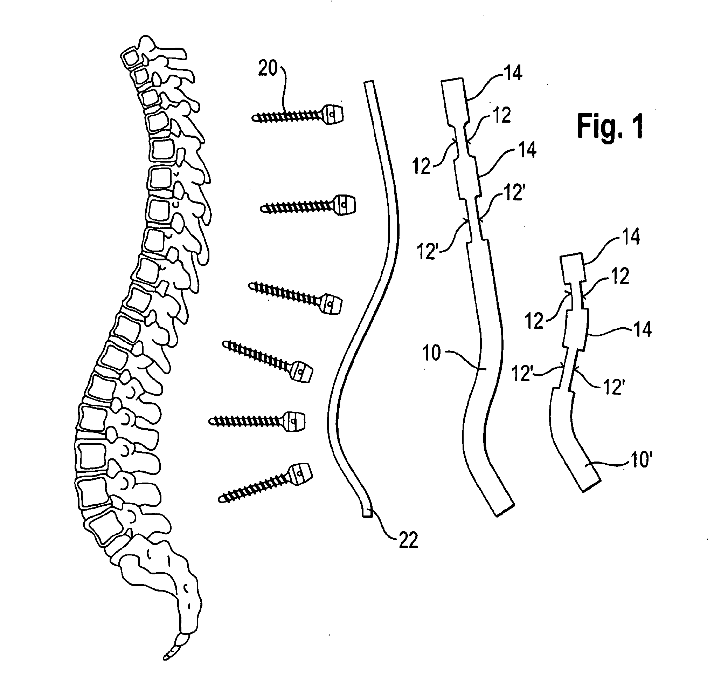

Fig. 1 shows in a schematic drawing an example of the spinal column, to which a dynamic stabilization device may be attached.

In a dynamic stabilization device, a flexible rod 22 may be employed to provide a fusion to vertebrae of the spinal column when the rod is clamped by respective bone anchoring devices 20. Thereby, the bone anchoring devices 20 are screwed in to specific vertebrae at appropriate height positions selected by the surgeon. The bone anchoring devices may be one of the monoaxial (i.e., the bone thread part and receiving part are rigidly fixed to each other) or polyaxial type (i.e., the bone thread part being pivotable with respect to an axis of receiving part prior to locking), but the present embodiments shall not be limited to the specific functions of the bone anchoring device.

The rod 22 which is schematically indicated in the center portion of Fig. 1 includes a constant diameter or thickness throughout its length and is made of an elastomer material. The thickness is chosen such as to provide a reasonable degree of stiffness or rigidity over its entire length. When this rod is clamped by corresponding receiving parts of the bone anchoring devices, a substantially rigid fusion between the vertebrae involved is achieved.

However, in various instances, it may be desirable to increase the flexibility of the rod. In one instance, the load which acts on vertebrae in a direct neighbourhood of fusioned parts of the spine may be too high. In order to relieve the load, the end part of the fusion can be provided with an enhanced degree of flexibility to enable a slight bending movement of the respective vertebrae.

Hence, in the present example, a rod 10 made of an elastomer material is manufactured from the rod 22, wherein the rod 10 now includes recesses 12 formed on opposite sides of the rod. The recesses are formed by removal of material from the rod 22. Due to this removal the rod is thinned, as a result of which the bending flexibility increases locally. The outermost vertebrae are thus allowed to perform a slight movement depending on the load.

The opposing recesses 12 are formed between two respective clamping sections 14 of the rod 10, which are defined such as to be received by the receiving parts of respective bone anchoring devices 20 and thus include an appropriate length section of the rod 10.

In order to allow further portions of the spine to undergo a slight bending movement, an additional pair of recesses 12' is formed in the rod surface. The opposing recesses 12' may leave a same thickness of the rod as the recesses 12, while the length may be slightly extended, as an example.

In Fig. 1 there is also shown a rod 10' as an alternative example which has the same features as the rod 10, but is provided with a comparatively shorter length.

The rod includes a preferably bio-compatibe elastomer material. Examples of such materials which may be embodied herein are polyurethane, polysiloxane, Poly(styrene-block-isobutylene-block-styrene) (SIBS), or polycarbonate urethane (PCU).

It may be noted that the term "rod" as used herein basically denotes a rod-shaped element, which may be provided as a single piece rod as well as a multi-part composite element, that is put together to yield a fusion. In the latter case, those parts may for example be provided with corresponding threads to connect the corresponding pieces. Further, one of the parts may include the elastomer material while another part of the rod may include, e.g., a metal.

A case in which for example a single piece rod is formed from injection molding of two or more different elastomer material components shall also be encompassed by the materials, wherein the recesses are applied afterwards.

It may be noted that whether a recess is formed by later removal of material, in particular punching, or not may be clearly recognizable from the product: due to a slight deformation, or flow of stressed elastomer material during punching and/or heating for example, plane surfaces or straight lines which are formed thereby also may become slightly concave or curved, respectively. Further, score marks or miniature rims may form across the cut surface along the punching direction.

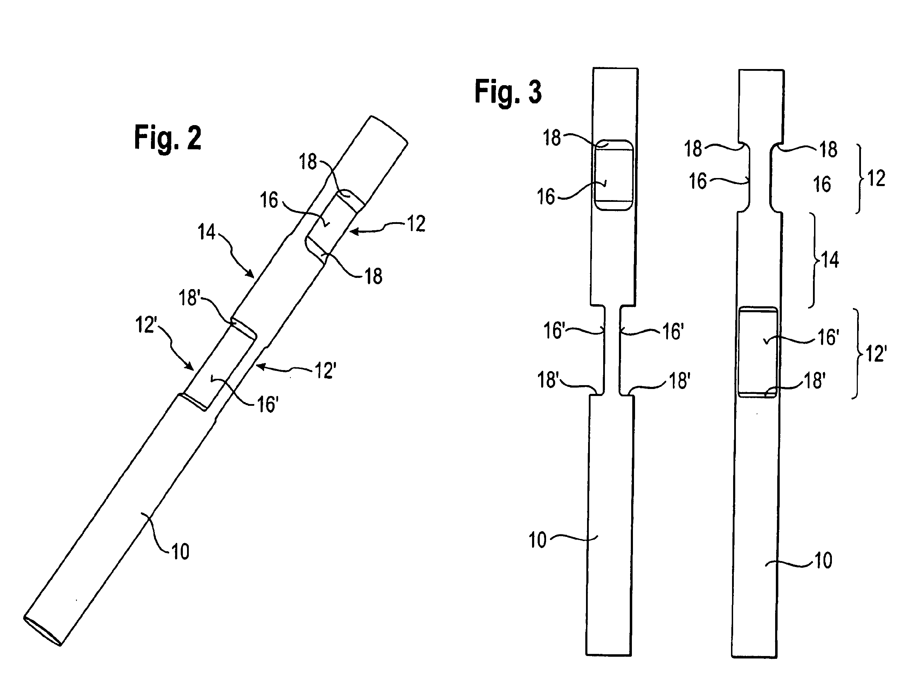

Figs. 2 and 3 show another example of a rod 10 which also includes an elastomer material similar to that shown in Fig. 1. However, unlike in the previous embodiment the recesses 12 and 12' (or pair of recesses) are oriented in different directions with respect to each other by about 90 degrees.

The recesses 12, 12' have a well-defined shape of limited length. As becomes evident from Fig. 3, the recesses 12 include a flat portion 16 and two rounded end portion 18. The further recesses 12' also have a flat portion 16', while the end portions 18' are steeper and rise up sharply. In this example, the flat portions 16' have a larger depth with respect to the surface than flat portions 16.

It may be noted that a rod 10 as described herein may also be formed with multiple recesses 12 or 12', all of which have the same shape - possibly with orientations with respect to the longitudinal axis of the rod, which differ from each other.

Despite the above mentioned presence of concave surfaces, score marks or miniature rims, it has been found that a punching operation may still fulfil the requirements with respect to surface roughness and punching accuracy quite satisfactorily, if a tool for punching a rod according to the invention is used.

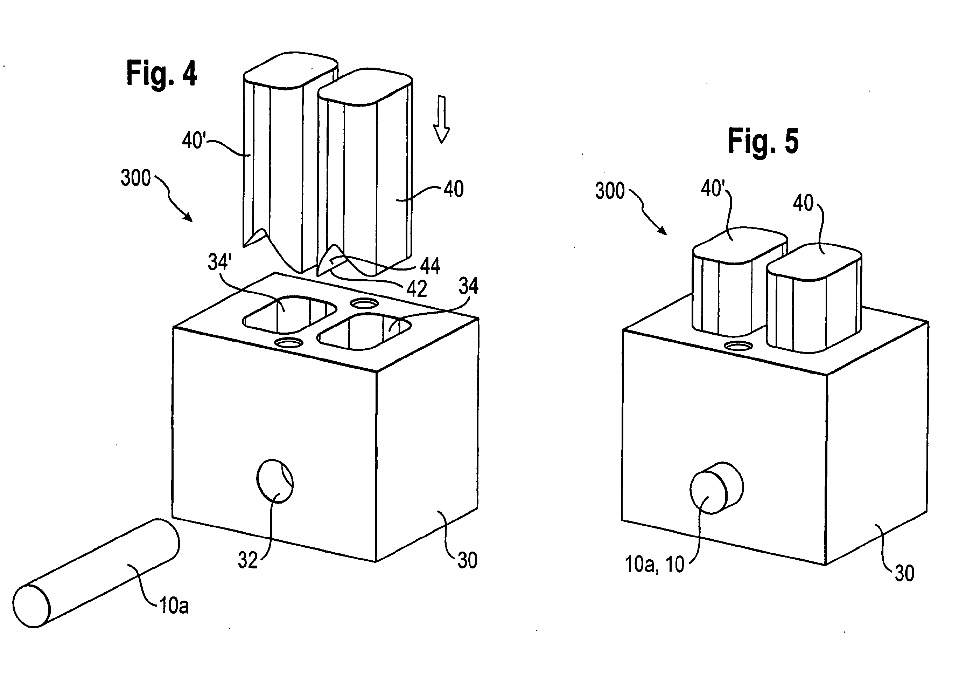

Figs. 4-7 show a first embodiment of a tool 300 for removing elastomer material from a rod 10a to manufacture one of the rods 10 shown in Figs. 1-3. The tool shown represents a punching tool. The tool comprises a socket die 30, which is provided with a bore 32 for insertion of the yet un-punched rod 10a, and with bores 34, 34' which receive each one of two punching presses 40, 40'. In this embodiment, the bore 32 extends through the socket die 30 in a horizontal direction while bore 34, 34' are oriented in a vertical direction. The rod 10a can be freely adjusted - i.e., pushed or rotated - in its position within bore 32 wherein the outer diameter of the rod 10a substantially corresponds to the inner diameter of the bore 32.

The punching presses 40, 40' are also shaped and sized to fit into the bores 34, 34'. As the bores 34, 34' are formed in parallel to each other, the orientation and guidance of the punching presses 40, 40' are also parallel. The bores 34, 34' intersect with the bore 32 such that the punching presses 40, 40' may cut material from rod 10a inserted in the bore 32. For this purpose, both presses are provided with cutting edges 42, 42' and cutting faces 44, 44' whose rake angle relative to the cutting direction serves to separate the removed elastomer material from the rod. The removed material may be discharged into a container not shown in the figures.

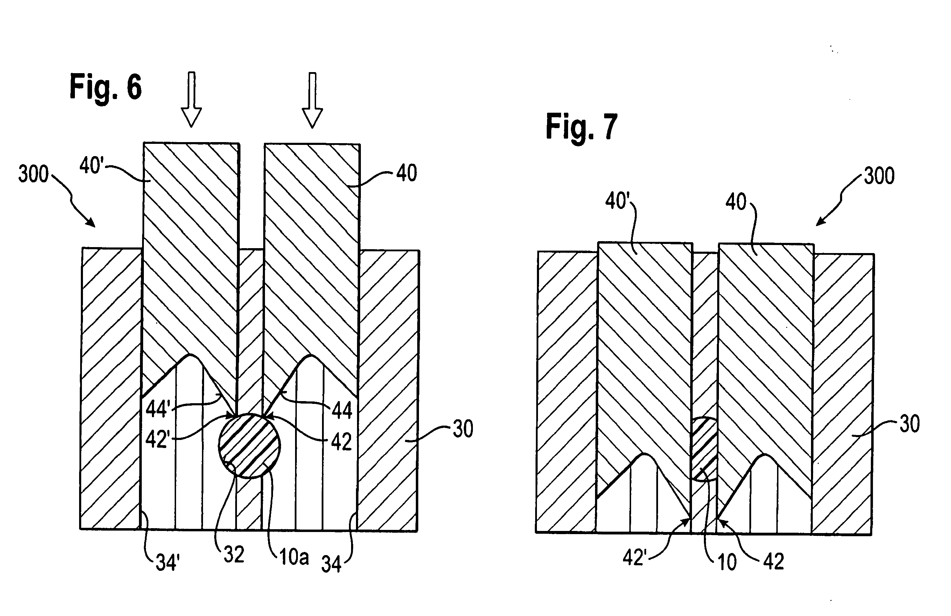

Upon exerting a load on the flexible rod, an inevitable deformation or flexible flow of material will occur in the rod. As depicted in the cross sections of Figs. 6 and 7, a simultaneous downward movement of the punching presses helps to develop a symmetric deformation of the flexible material of the rod during the punching operation. Since bores 34, 34' intersect with the bore 32 off-center from its longitudinal axis on opposite sides thereof, recesses such as shown in Figs. 1-3 may be formed in the rod surface. Both punching presses may therefore be mechanically coupled with each other and further with an operating means. Such operating means may for example be a simple toggle joint. However, any other operating means capable of pressing down the punching presses with suitable force may be employed as well. It is clear, that cutting tools including one or more punching presses may be used.

The separating tool may advantageously be placed or installed in the vicinity of surgeon's operating site, i.e., in a hospital. Accordingly, the surgeon or an attending person may in situ decide where and to what extent recesses shall be applied to the rod.

Hence, costs and efforts can be reduced which are necessary to provide a flexible stabilization device suited for the specific needs of a patient.

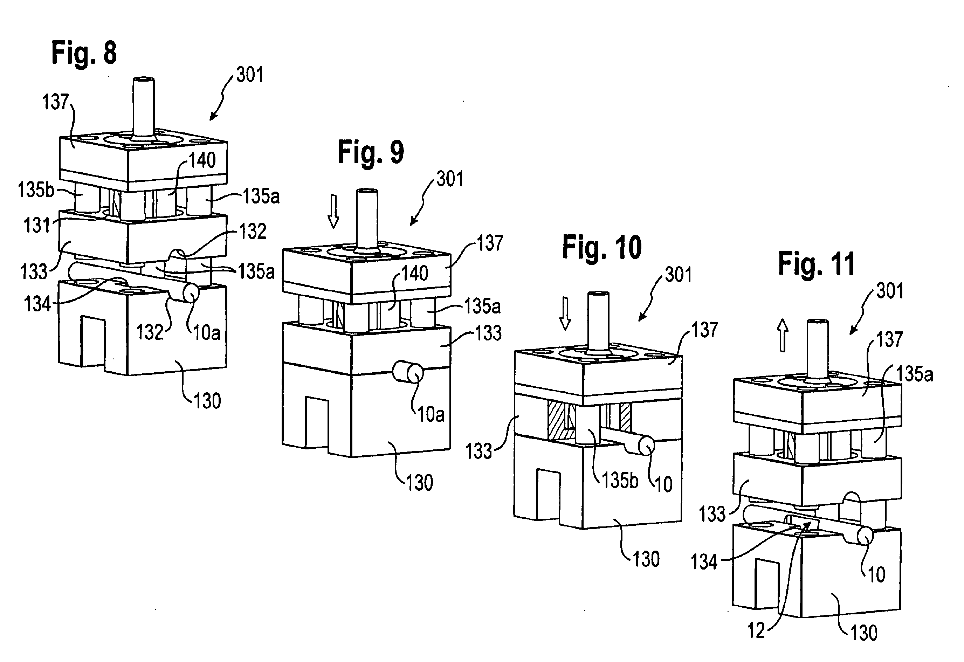

Figs. 8-11 show another embodiment of a tool 301 for removing material from a rod 10a. This embodiment differs from the previous embodiment particularly in that the tool includes a structure which is substantially composed of three plates 130, 133, 137. Other parts not described in detail herein - in particular the shape, orientation and function of the presses - are the same as in the embodiment of Figs. 4-7.

The bottom die plate 130 includes one half of a bore 132 designed to receive the yet un-punched rod 10a. A presser plate 133 is supported by a first spring means (e.g., a coil spring not shown) in a distance above the bottom die plate 130 and includes the other half part of the bore 132 in a bottom face thereof. When the presser plate 133 moves down until the upper face of the bottom die plate 130 is contacted, the rod 10a is held fixed inside the bore 132.

Two guide rods 135a extend upwards from the bottom die plate 130. The presser plate 133 has corresponding bores which receive the guide rods such that the presser plate 133 is held to be movable up and down along the guide rods 135a. The die plate 130 and the presser plate 133 correspond to the socket die of the previous embodiment.

A cutting plate 137 supported in a distance above the presser plate is also guided by the guide rods 135a. The distance between plates 133, 137 is maintained by a second spring mechanism whose spring force is larger than that of the first spring means. Two further guide rods 135b extend downward from the cutting plate 137 in order to be received by corresponding bores of presser plate 133. The presser plate 133 is also movable with respect to the guide rods 135b.

The cutting plate 137 further has - similar to the previous embodiment - two cutting presses extending downward through an opening 131 of the presser plate 133 towards corresponding bores 134 formed in the bottom die plate 130. Bores 132 and 134 intersect each other as in the embodiment shown in Figs. 6-7.

The tools shown in Figs. 4-11 may be fabricated from stainless steel or other suitable materials.

A method of manufacturing a rod 10 as shown in Figs. 1-3 using the tool as described above is now explained with reference to Figs. 8-11:
First, as shown in Fig. 8, the tool is in an uncompressed state, wherein the rod 10a can be inserted into the opened bore 132 on a top face of the bottom die plate 130.
Next, as shown in Fig. 9, the cutting plate 137 is moved down using, e.g. a toggle joint, etc. Since the second spring means requires a larger force to be compressed than the first spring means, the presser plate coincidently moves down along the guide rods 135a until the presser plate contacts the bottom die plate 130 such that the rod 10a is fixed inside the bore 132.
Next, as shown in Fig. 10, the cutting plate is further moved down also against the force of the second spring means. Eventually, the cutting presses 140 along their travel through the bores 134 hit the rod 10a (which is now held fixed by the presser plate) and remove an amount of elastomer material thereof.
Next, as shown in Fig. 11, the load manually exerted on the cutting plate 137 is removed such that the tool 301 returns to the uncompressed state, leaving behind a rod 10 which includes recesses 12. In order to apply further recesses 12 or 12', the rod can then be newly positioned, i.e., shifted and/or rotated.

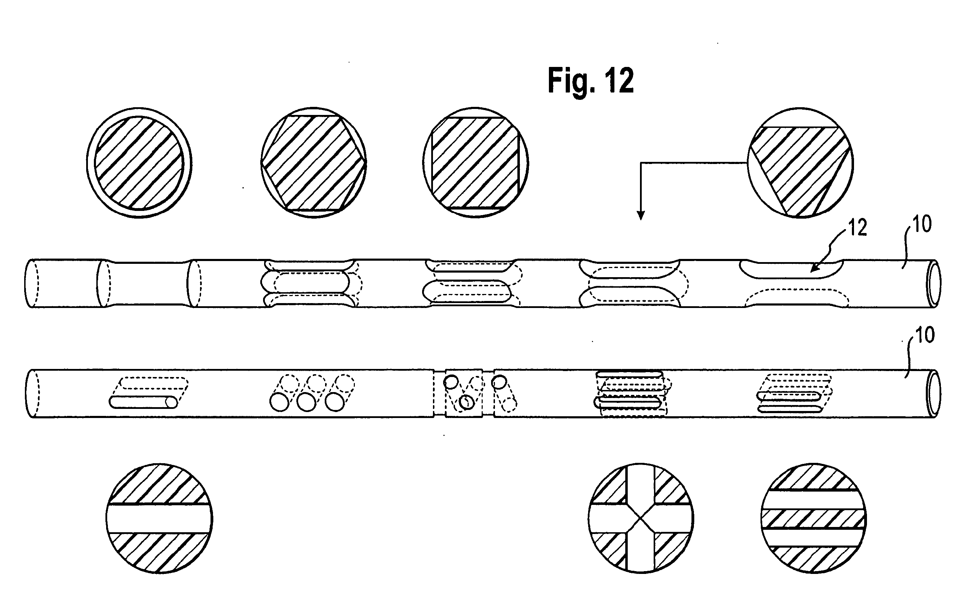

The shapes and sizes of recesses formed in the rod by removing material are not limited to those examples shown in the embodiments above. As shown in Fig. 12, which indicates various kinds of recesses in perspective view along with the associated cross sections of the rod, the recesses may be formed by removing material (a) from the periphery of the rod (upper section of Fig. 12) to yield grooves, or (b) from inner through holes extending through a rod (bottom section of Fig. 12).

The rod can have any cross-section in sections other than those comprising the recesses, e.g., cylindrical, hexagonal, square, etc.

Recesses are applied to a rod of a flexible stabilization device by removal of material in selected areas. Hence, clamping sections can be maintained between the recesses which may serve to be clamped by bone anchoring devices. The desired bending properties of the rod are thus concentrated in these selective areas outside the clamping sections. As a result, conventional abrasion of elastomer material due to the grinding of a bending rod surface inside a rigid receiving part can be considerably reduced. Consequently, the endurance of the rod can be prolonged.

## Claims

1. A tool (300; 301) for separating material from a flexible rod-shaped element (22, 10a), which includes an elastomer material, in order to provide at least one first recess (12) in the surface of the rod-shaped element, the tool comprising:
a socket die (30; 130, 133) having:
a first bore (32; 132) for receiving the rod-shaped element (22, 10a), and
at least one second bore (34; 134) for receiving a first punching press (40; 140); and
a first punching press, that fits within the at least one second bore being moveable with respect thereto; **characterized in that**
the at least one second bore (34; 134) intersects with the first bore (32; 132) off-center from its longitudinal axis to enable punching out material from the rod-shaped element to form the recess (12) due to the movable first punching press (40; 140).

2. The tool (300; 301) according to claim 1, further comprising a second punching press (40'; 140') and a third bore (34; 134') for receiving the second punching press, the second punching press being movable within the third bore, wherein both the second and the third bores are arranged in parallel and partially intersect with the first bore at positions on opposing sides of the first bore.

3. The tool (300; 301) according to claim 2, wherein the first and second punching presses (40, 40'; 140, 140') are connected with each other such as to provide a common punching movement.

4. The tool according to one of claims 1 to 3, further comprising a toggle joint mechanism to operate the movement of the first inner punching press, or the first and second inner punching presses, respectively.

## Patentansprüche

1. Ein Gerät (300; 301) zum Trennen eines Materials von einem flexiblen stabförmigen Element (22, 10a), welches ein Elastomermaterial umfasst, um in der Oberfläche des stabförmigen Elements wenigstens eine erste Ausnehmung (12) einzurichten, wobei das Gerät umfasst:
ein Stanzlager (30; 130, 133), aufweisend:
Eine erste Bohrung (32; 132) zum Aufnehmen des stabförmigen Elements (22, 10a), und
wenigstens eine zweite Bohrung (34; 134) zum aufnehmen einer ersten Stanzpresse (40; 140); und
eine erste Stanzpresse, die innerhalb der wenigstens einen zweiten Bohrung bewegbar relativ zu dieser eingepasst ist; **dadurch gekennzeichnet, dass**
die wenigstens eine zweite Bohrung (34; 134) sich mit der ersten Bohrung (32; 132) dezentral von deren Längsachse schneidet, um ein Ausstanzen von Material von dem stabförmigen Element zu erlauben, wobei die Ausnehmung (12) mit Hilfe der bewegbaren ersten Stanzpresse (40; 140) ausgebildet wird.

2. Das Gerät (300, 301) gemäß Anspruch 1, ferner umfassend eine zweite Stanzpresse (40'; 140') und eine dritte Bohrung (34; 134') zum Aufnehmen der zweiten Stanzpresse, wobei die zweite Stanzpresse innerhalb der dritten Bohrung bewegbar ist, wobei sowohl die zweite als auch die dritte Bohrung parallel zueinander angeordnet sind, und sich teilweise mit der ersten Bohrung an Positionen auf gegenüberliegenden Seiten der ersten Bohrung schneiden.

3. Das Gerät (300; 301) gemäß Anspruch 2, wobei die ersten und zweiten Stanzpressen (40, 40'; 140, 140') miteinander verbunden sind, um eine gemeinsame Stanzbewegung zu ermöglichen.

4. Das Gerät gemäß allen der Ansprüche 1 bis 3, ferner umfassend einen Kniehebelmechanismus, um die Bewegung der ersten inneren Stanzpresse, oder der ersten und zweiten inneren Stanzpressen in entsprechender Weise, zu betreiben.

## Revendications

1. Outil (300 ; 301) destiné à séparer un matériau à partir d'un élément en forme de tige flexible (22, 10a), qui comporte un matériau élastomère, afin de fournir au moins un premier évidement (12) dans la surface de l'élément en forme de tige, l'outil comprenant :
une matrice de support (30 ; 130, 133) ayant :
un premier alésage (32; 132) destiné à recevoir l'élément en forme de tige (22, 10a), et
au moins un deuxième alésage (34 ; 134) destiné à recevoir une première presse à découper (40 ; 140) ; et
une première presse à découper, qui est ajustée dans l'au moins un deuxième alésage étant mobile par rapport à celui-ci ; **caractérisé en ce que**
l'au moins un deuxième alésage (34 ; 134) se croise avec le premier alésage (32 ; 132) de manière décentrée de son axe longitudinal pour permettre le découpage d'un matériau à partir de l'élément en forme de tige afin de former l'évidement (12) grâce à la première presse à découper mobile (40 ; 140).

2. Outil (300; 301) selon la revendication 1, comprenant en outre une deuxième presse à découper (40' ; 140') et un troisième alésage (34 ; 134') destiné à recevoir la deuxième presse à découper, la deuxième presse à découper étant mobile dans le troisième alésage, où les deuxième et troisième alésages sont disposés en parallèle et se croisent partiellement avec le premier alésage au niveau des positions sur des côtés opposés du premier alésage.

3. Outil (300 ; 301) selon la revendication 2, dans lequel les première et deuxième presses à découper (40, 40' ; 140, 140') sont reliées l'une à l'autre de manière à fournir un mouvement de découpage commun.

4. Outil selon l'une des revendications 1 à 3, comprenant en outre un mécanisme d'articulation à genouillère afin d'actionner le mouvement de la première presse à découper intérieure, ou des première et deuxième presses à découper intérieures, respectivement.
